(19) Europäisches Patentamt
European Patent Office
Office européen des brevets

(11) EP 4 567 502 A1

(12) **EUROPEAN PATENT APPLICATION**

(43) Date of publication:
**11.06.2025 Bulletin 2025/24**

(21) Application number: **23307134.9**

(22) Date of filing: **05.12.2023**

(51) International Patent Classification (IPC):
**G02C 11/00** (2006.01)   **A61B 5/00** (2006.01)
**G06F 1/16** (2006.01)   **G02B 27/01** (2006.01)
**G06F 1/3231** (2019.01)

(52) Cooperative Patent Classification (CPC):
**G02C 11/10; A61B 5/6803; A61B 5/7257;
G06F 1/163; G06F 1/1694; G06F 1/3231;
G06F 1/3287; G02B 2027/0178**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Essilor International
94220 Charenton-Le-Pont (FR)**

(72) Inventors:
• **AMIR, Bruno
   94100 Saint-Maur-des-Fosses (FR)**
• **GIL, Paul
   80000 Amiens (FR)**
• **SAHLER, Jean
   92160 Antony (FR)**

(74) Representative: **Korakis-Ménager, Sophie
   Essilor International
   Propriété Intellectuelle
   147, rue de Paris
   94220 Charenton-le-Pont (FR)**

(54) **DEVICE, HEAD-MOUNTABLE DEVICE AND EYEWEAR INTENDED TO BE WORN BY A USER AND CONFIGURED FOR DETERMINING IF THE USER IS WEARING THE DEVICE**

(57)    Device, head-mountable device and eyewear intended to be worn by a user. The device is configured for determining if the user is wearing the device. The device is configured to obtain a signal representing vibrations produced by the user, to determine, in a predetermined frequency range, a power spectrum of the signal, to determine, based on the power spectrum, if the user is wearing the device. The predetermined frequency range is from 8 Hz to 12 Hz.

[Fig. 6a]

EP 4 567 502 A1

## Description

## Technical field

This disclosure relates to devices intended to be worn by a user and configured for determining if the user is wearing the device and methods for determining when the device, intended to be worn by the user, is worn by the user.

## Background information and prior art

For some device, for example eyewear, the worn detection allows an improved functioning of the device. For example, the device may be configured to provide a monitoring of values of physiological parameters or may provide to the user different functions. For example, the device may comprise audio functions and when the device is an eyewear, the eyewear may comprise active optical devices.

An accurate detection of the wearing of the device by the user allows an activation of these functions only when the device is worn by the user i.e., when the user may use the functions. The activation of the functions only when the user may use the functions allows a reduction of the energy consumed by the device.

An accurate detection of the wearing of the device by the user allows a monitoring only when the device is worn by the user. Realizing the monitoring only when the device is worn by the user allows an accurate determination of the values of the physiological parameters.

There is a need for devices intended to be worn by a user and configured for determining if the user is wearing the device and methods for determining when the device, intended to be worn by the user, is worn by the user.

## Summary

The following presents a simplified summary to provide a basic understanding of various aspects of this disclosure. This summary is not an extensive overview of all contemplated aspects and is intended to neither identify key or critical elements of all aspects nor delineate the scope of any or all aspects. The sole purpose is to present some concepts of one or more aspects in a simplified form as a prelude to the more detailed description that is presented later.

One aspect of this disclosure is a device. The device is intended to be worn by a user. The device is configured for determining if the user is wearing the device. The device is configured to obtain a signal representing vibrations produced by the user, to determine, in a predetermined frequency range, a power spectrum of the signal, to determine, based on the power spectrum, if the user is wearing the device. The predetermined frequency range is from 8 Hz to 12 Hz.

The device of this disclosure avoids giving false positive, especially compared using sensors for detecting the proximity of the device with the user. like capacitive or optical sensors.

The device of this disclosure may discriminate the nearby object from human body to avoid false positive.

Another aspect of this disclosure is a head-mountable device. The head-mountable device is configured for determining if the user is wearing the head-mountable device. The head-mountable device is configured to obtain a signal representing vibrations produced by the user, to determine, in a predetermined frequency range, a power spectrum of the signal, to determine, based on the power spectrum, if the user is wearing the head-mountable device. The predetermined frequency range is from 8 Hz to 12 Hz.

Another aspect of this disclosure is an eyewear. The eyewear is configured for determining if a user is wearing the eyewear. The eyewear is configured to obtain a signal representing vibrations produced by the user, to determine, in a predetermined frequency range, a power spectrum of the signal, to determine, based on the power spectrum, if the user is wearing the eyewear. The predetermined frequency range is from 8 Hz to 12 Hz.

Another aspect of this disclosure is a computer implemented method for determining when a device, intended to be worn by a user, is worn by the user. The method comprises obtaining a signal representing vibrations produced by the user, determining, in a predetermined frequency range, a power spectrum of the signal, determining, based on the power spectrum, if the user is wearing the device. The predetermined frequency range is from 8 Hz to 12 Hz.

Another aspect of this disclosure is a computer program comprising instructions which, when the computer program is executed by a computer, cause the computer to carry out a computer-implemented method for determining when a device, intended to be worn by a user, is worn by the user. The computer-implemented method comprises obtaining a signal representing vibrations produced by the user, determining, in a predetermined frequency range, a power spectrum of the signal, determining, based on the power spectrum, if the user is wearing the device. The predetermined frequency range is from 8 Hz to 12 Hz.

A computer may include a memory and a processor. Examples of processors include microprocessors, microcontrollers, graphics processing units (GPUs), central processing units (CPUs), application processors, digital signal processors (DSPs), reduced instruction set computing (RISC) processors, systems on a chip (SoC), field-programmable gate arrays (FPGAs), programmable logic devices (PLDs), state machines, gated logic, discrete hardware circuits, and other suitable hardware configured to perform the various functionality described throughout this disclosure. The memory may be a computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor of the computer.

Another aspect of this disclosure is a non-transitory program storage device, readable by a computer, tangibly embodying a program of instructions executable by the computer to perform a computer-implemented method for determining when a device, intended to be worn by a user, is worn by the user. The computer-implemented method comprises obtaining a signal representing vibrations produced by the user, determining, in a predetermined frequency range, a power spectrum of the signal, determining, based on the power spectrum, if the user is wearing the device. The predetermined frequency range is from 8 Hz to 12 Hz.

## Description of the drawings

For a more complete understanding of the description provided herein and the advantages thereof, reference is now made to the brief descriptions below, taken in connection with the accompanying drawings and detailed description, wherein like reference numerals represent like parts.

Figure 1 represents the device intended to be worn by a user.

Figure 2 represents an eyewear.

Figure 3 represents a removable clip attachable to the eyewear.

Figure 4 represents an augmented reality eyewear.

Figure 5 represents the method for determining if the user is wearing the device.

Figures 6-a and 6-b represent for two situations of a first use case, the signal representing vibrations received by the module and the power spectrum of this signal.

Figures 7-a and 7-b represent for two situations of a second use case, the signal representing vibrations received by the module and the power spectrum of this signal.

The figure 8 represents, in a third use case, the signal representing vibrations received by the module and the power spectrum of this signal.

The figure 9 represents, in a fourth use case, the signal representing vibrations received by the module and the power spectrum of this signal.

The figure 10 represents, in a fifth use case, the signal representing vibrations received by the module and the power spectrum of this signal.

The figure 11-a and 11-b represents for two situations of a sixth use case the signal representing vibrations received by the sensor and the power spectrum of this signal.

## Detailed description of embodiments

The detailed description set forth below in connection with the appended drawings is intended as a description of various possible embodiments and is not intended to represent the only embodiments in which the concepts described herein may be practiced. The detailed description includes specific details for the purpose of providing a thorough understanding of various concepts. However, it will be apparent to those skilled in the art that these concepts may be practiced without these specific details. In some instances, well-known structures and components are shown in block diagram form to avoid obscuring such concepts.

### *Description of the device intended to be worn by the user and configured for determining if the user is wearing the device*

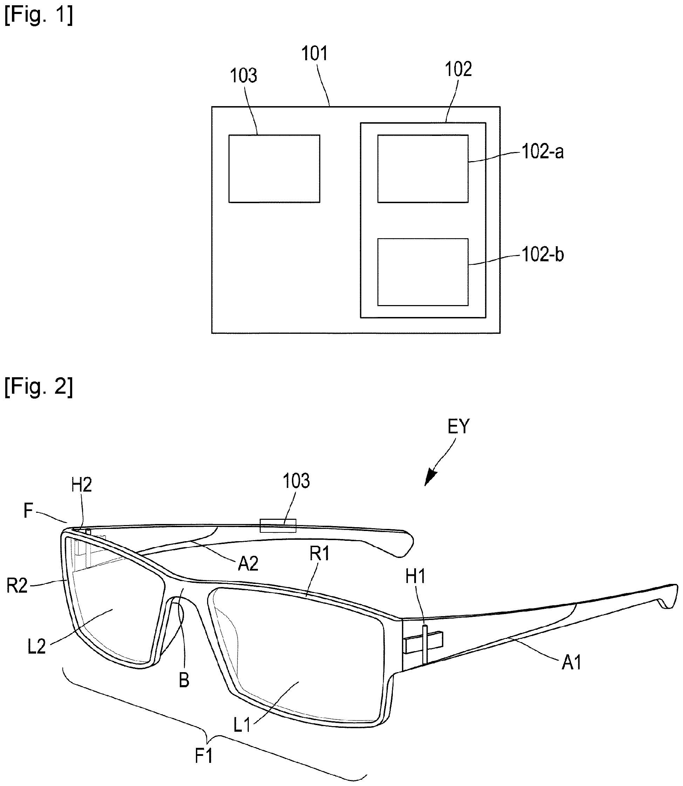

Figure 1 represents a device 101 intended to be worn by the user and configured for determining if the user is wearing the device. The device 101 comprises a calculation module 102 and a sensor 103 for generating a signal representing vibrations of the user. The calculation module 102 is configured to determine when the device 101 is worn by the user.

The vibrations may be produced by muscles of the user.

The calculation module 102 comprises a memory 102-a and a processor 102-b.

The calculation module may be a low resource calculation module.

Examples of processors 102-b include microprocessors, microcontrollers, graphics processing units (GPUs), central processing units (CPUs), application processors, digital signal processors (DSPs), reduced instruction set computing (RISC) processors, systems on a chip (SoC), baseband processors, field-programmable gate arrays (FPGAs), programmable logic devices (PLDs), state machines, gated logic, discrete hardware circuits, and other suitable hardware configured to perform the various functionality described throughout this disclosure.

The memory 102-a is computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor 102-a of the calculation module 102.

The device 101 may comprises other modules. The calculation module 102 may be included in an independent module. The calculation module 102 may also be part of one of the other modules of the device 101.

The device 101 may be a head-mountable device.

The figure 2 represents eyewear EY, which are an example of head-mountable device. This eyewear EY comprise two lenses L1 and L2 and a frame F. The frame F comprises two arms or temples A1 and A2 and a front part F1. The front part F1 comprises a right rim R1 and left rim R2 linked together by a bridge B. The front part F1 and the two arms A1 and A2 are linked using two hinges H1 and H2. The hinges H1 and H2 allow the individual to fold the arms A1 and A2 along the front part F1. The rims R1 and R2 of the frame F1 are configured to receive and to maintain the lenses L1 and L2. The eyewear EY can be prescription eyewear for which the refraction has been adapted to the individual by an eye care professional and eyewear without refraction for example sunglasses.

As represented in the figure 2, the eyewear EY may integrate the sensor 103 for determining a signal representing vibrations produced by the muscles of the user. The sensor 103 may be located in one or both of the arms A1 and A2.

In embodiments the calculation module 102 may be integrated in the eyewear EY.

In other embodiments the calculation module 102 may be a module independent of the eyewear EY. In this case the calculation module 102 may be for example a smartphone.

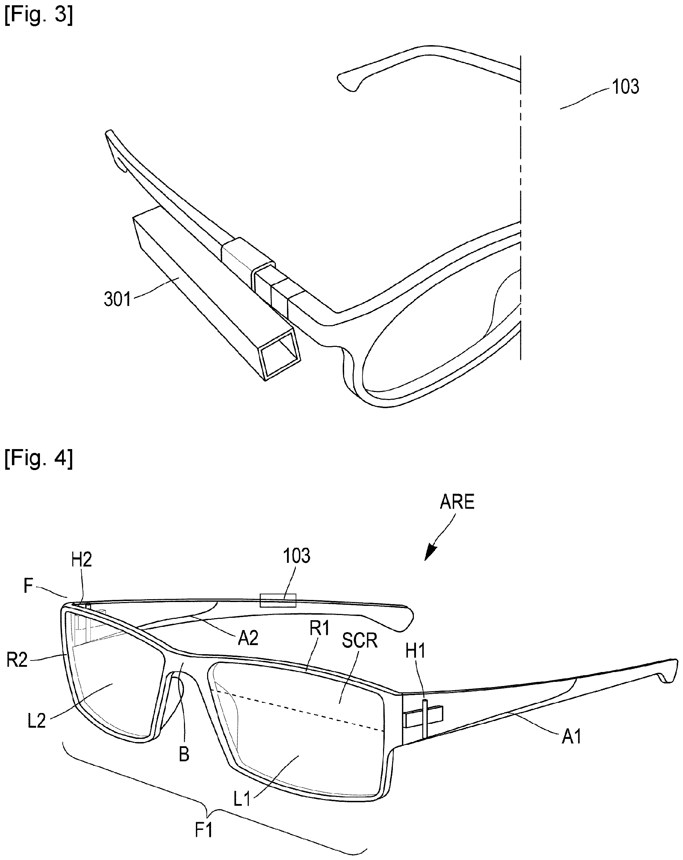

In other embodiments, represented figure 3, a removable clip 301 may be attached to the eyewear EY. This removable clip 301 may comprise the sensor 103 and possibly the calculation module 102.

The eyewear EY may also comprise an optical device. This optical device may be an active optical device.

The active optical device may be an electrochromic optical lens or may be configured to display elements. To display elements, the active optical elements may comprise a Light Optical Element (LOE).

By an active optical device, one means an optical device in which a value of a parameter of a functionality of the optical device can be modified on-demand. The value of the parameter may be modified by applying a signal, for example an electrical signal to terminals of the active optical device. The functionality may be one of the following:

- a transmittance level of the active optical device,
- a tint of the active optical device,
- a reflectance level of the active optical device and
- a parameter of a polarization of the active optical device, for example a polarization angle of the active optical device,
- an optical power of the active optical device,

- elements displayed by the active optical device. The elements may be images, symbols, letters, or texts. In embodiments, the elements to be displayed and the position of these elements may be selected.

In embodiments the functioning of the active optical device may depend on whether one detects that the user is wearing the device.

The figure 4 represents augmented reality eyewear ARE, which are another example of head-mountable device. This augmented reality eyewear ARE comprises also two lenses L1 and L2 and a frame F. The frame F comprises two arms or temples A1 and A2 and a front part F1. The front part F1 comprises a right rim R1 and left rim R2 linked together by a bridge B. The front part F1 and the two arms A1 and A2 are linked using two hinges H1 and H2. The hinges H1 and H2 allow the individual to fold the arms A1 and A2 along the front part F1. The rims R1 and R2 of the frame F1 are configured to receive and to maintain the lenses L1 and L2. Furthermore, the augmented reality eyewear ARE comprises a screen SCR, generally a see-through display or transparent display.

As represented in the figure 4, the augmented reality eyewear ARE may integrates the sensor 103 for determining a signal representing vibrations produced by the muscles of the user. The sensor 103 may be located in one or both of the arms A1 and A2.

As for the eyewear EY, in embodiments the calculation module 102 may be integrated in the augmented reality eyewear ARE.

As for the eyewear EY, in other embodiments, the calculation module 102 may be a module independent of the augmented reality eyewear ARE. In this case the calculation module 102 may be for example a smartphone.

As for the eyewear EY, in other embodiments, the removable clip 301 may be attached to the augmented reality eyewear ARE.

As for the eyewear EY, in other embodiments, the augmented reality eyewear ARE may also comprise an optical device. This optical device may be an active optical device.

In embodiments the functioning of the augmented reality eyewear ARE may depend on whether one detects that the user is wearing the augmented reality eyewear ARE.

The device 101 may also be a watch, more precisely a wristwatch. Wristwatches are designed to be worn around the wrist, attached by a watch strap or other type of bracelet, including metal bands, leather straps, or any other kind of bracelet. In this case the watch may include the sensor 103 and possibly the calculation module 102.

The watch may also be a smartwatch that is a wearable computer in the form of a watch. Modern smartwatches provide generally a local touchscreen interface for daily use, while an associated smartphone application and may comprise sensors used to determine physiological parameters of the individual. In this case the sensor 103 may be one of these sensors. The calculation module 102 may be included in the smartwatch and may be share with other functions of the smartwatch.

In embodiments the functioning of the watch may depend on whether one detects that the user is wearing the watch.

The sensor 103 may be excited by the vibrations of the muscles of the user. The sensor 103 of the device 101 may be a MEMS sensor chosen among:

- an accelerometer
- a capacitive sensor

Using a MEMS sensor allows the sensor to be cheap and widely available.

Using the accelerometer is advantageous because accelerometers are cheap widely available and in some embodiments are integrated in eyewear to realize other functions.

### Description of the method for determining if the user is wearing the device

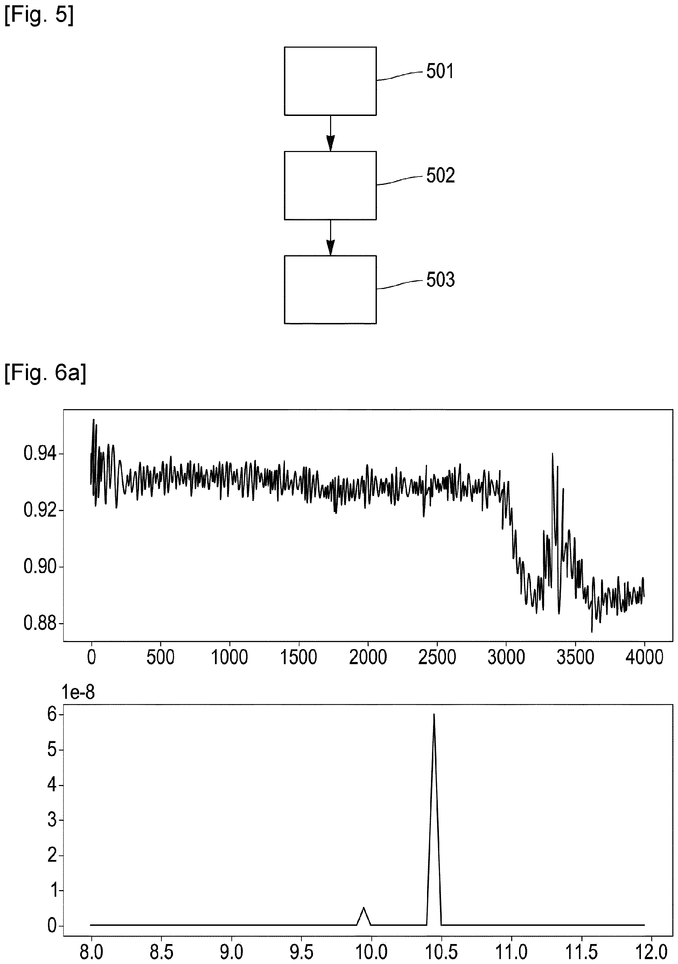

To determine whether the user is wearing the device 101, the memory 102-a may store a computer program comprising instructions which, when the program is executed by the processor 102-b, cause the calculation module 102 to carry out a method, for example a computer implemented method, for determining if the user wears the device 101. The method, as presented in figure 5, comprises:

- a step 501 obtaining a signal representing vibrations produced by the user,
- a step 502 determining, in a predetermined frequency range, a power spectrum of the signal,
- a step 503 determining, based on the power spectrum, if the user is wearing the device.

The predetermined frequency range is from 8 Hz to 12 Hz.
The vibrations may be produced by muscles of the user.
The method of this disclosure has the advantage of being implementable on a low resource calculation module and the time to detect a worn event is about few seconds and depends mainly on the time necessary to acquire the signal representing the vibrations.
By a power spectrum of a signal one means a description of a distribution of a power, of the signal, into frequency components composing the signal.
The autocorrelation of a real, stationary signal $x(t)$ is defined by:

$$R_x(\tau) = E\{x(t)\,x(t+\tau)\}$$

Where $E\{.\}$ is the expected value operator and $\tau$ is the time lag.

The power spectral density (PSD) or power spectrum of a stationary signal is defined as the Fourier transform of the autocorrelation function.

$$PSD(f) = \int_{-\infty}^{\infty} R_x(\tau)e^{-i2\pi f \tau}d\tau$$

The PSD of a signal represents the distribution of the power as a function of the frequency.
During the step 501, the signal is obtained from the sensor 103 of the device 101.
The method of the figure 5 may comprise the detection of peaks in the power spectrum and the step 503 may comprise the determination that the user is wearing the device when at least one peak is detected.
The method of the figure 5 may comprise, for detecting the peaks, a comparison of values of the power spectrum to a predetermined level and to detect a peak when one of the values of the power spectrum is above the predetermined level.
The predetermined level may be determined by using a 99% confidence method.
The 99% confidence method involves using the same measured values of the signal, but considering them in a random sequence, to disrupt the periodicity of the signal, aiming solely to mitigate the noise. This can be conducted repeatedly to mitigate the risk of inadvertently recreating incorrect sequences. The 99% confidence method is mainly suitable to be used during a calibration phase, with the aim of determining the noise of the sensor 103.
The method of the figure 5 may comprises, for determining that the user is wearing the device, a determination of a signal-to-noise ratio of the power spectrum. The method may further comprise a comparison of the signal-to-noise ratio with a predetermined signal-to-noise ratio threshold. When the signal-to-noise ratio is above the predetermined signal-to-noise ratio threshold one determines that the user is wearing the device.
The predetermined signal-to-noise ratio threshold may be 0 dB.
By signal-to-noise ratio of a signal one means a measure that compares a level of a desired signal, inside the signal, to a level of a background noise comprised in the signal. The signal-to-noise ratio may be defined as the ratio of a power of the desired signal to a power of the background noise. This ratio may be expressed in decibels. A ratio higher than 1 (greater than 0 dB) indicates the level of the desired signal is above the level of the background noise.
One may determine the signal-to-noise ratio by determining a ratio between a mean deviation of the power spectrum and a standard deviation of the power spectrum.
The mean deviation of the power spectrum may be determined by using the following formula:

$$M = \frac{\sum_{n=1}^{N} |x_n - \mu|}{N}$$

Where:

*M* is the mean deviation
*N* is the number of values of the power spectrum signal
$x_n$ is the value reference *n* of the power spectrum signal
$\mu$ is a mean value of the values of the power spectrum signal

The standard deviation of the power spectrum may be determined by using the following formula:

$$SD = \sqrt{\frac{\sum_{n=1}^{N} (x_n - \mu)^2}{N}}$$

Where:

*SD* is the standard deviation
*N* is the number of values of the power spectrum signal
$x_n$ is the value reference *n* of the power spectrum signal
$\mu$ is a mean value of the values of the power spectrum signal

The mean value of the power spectrum signal may be determined by using the following formula:

$$\mu = \frac{\sum_{n=1}^{N} x_n}{N}$$

Where:

*N* is the number of values of the power spectrum signal
$x_n$ is the value reference *n* of the power spectrum signal
$\mu$ is the mean value of the values of the power spectrum signal

Using the signal-to-noise ratio and the peak signal-to-noise ratio allows a better detection of the wearing of the device 101 in complex situations, for example situations involving dynamic activities (with a lot of motion), like frame in the backpack or on the nose of a walking user.

A more complex way is to use a kaiser windowed periodogram. The peak of the fundamental frequencies are detected, and the power is computed using harmonic frequencies. The rest of the signal is considered as noise.

The method of the figure 5 may comprises, for determining that the user is wearing the device, a determination of a peak signal-to-noise ratio of the power spectrum. The method may further comprise a comparison of the peak signal-to-noise ratio with a predetermined peak signal-to-noise ratio threshold. When the peak signal-to-noise ratio is above the predetermined peak signal-to-noise ratio threshold one determines that the user is wearing the device. The predetermined peal signal-to-noise ratio threshold may be 13 dB.

By peak signal-to-noise ratio of a signal one means a measure that compares a level of a maximum value of a desired signal, inside the signal, to a level of a background noise comprised in the signal. The peak signal-to-noise ratio may be defined as the ratio of a power of the maximum value of the desired signal power to a power of the background noise. This ratio may be expressed in decibels. A ratio higher than 1 (greater than 0 dB) indicates the level of the maximum value of the desired signal is above the level of the background noise.

To determine when to use the threshold power spectrum criteria or the signal-to-noise ratio criteria (or peak signal-to-noise ratio criteria) one may use indications provided by the sensor 103 indicating if the user is immobile or active. If the sensor 103 indicates that the user is active one may advantageously use the signal-to-noise ratio criteria (or peak signal-to-noise ratio criteria).

To determine the same, one may otherwise use the signal received by the sensor 103. Determining whether the user is static or moving may be realized by using the standard deviation of the signal and a threshold. Advantageously, one may determine that the user is moving when the standard deviation is above 0.07 g. Otherwise, the subject is immobile. If using this method, one detects that the user is active one may advantageously use the signal-to-noise ratio criteria (or peak signal-to-noise ratio criteria).

In embodiments, the method of figure 5 could be realized a plurality of times to confirm whether the user is wearing the device 101.

### Description of use cases

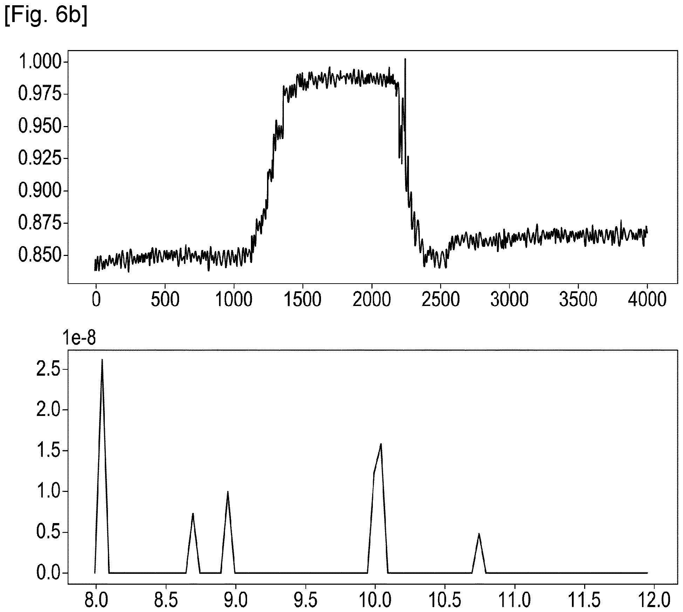

In a first use case, the device 101 is an eyewear, and the user is wearing the eyewear in a regular way (on the nose). The user is immobile. The figures 6-a and 6-b represent in two situations of the first use case, the signal representing vibrations received by the sensor 103 (top part) and the power spectrum of this signal (bottom part).

In both situations, the power spectrum of the signal has been threshold. In both cases peaks have been detected and one determines that the eyewear is worn by the user.

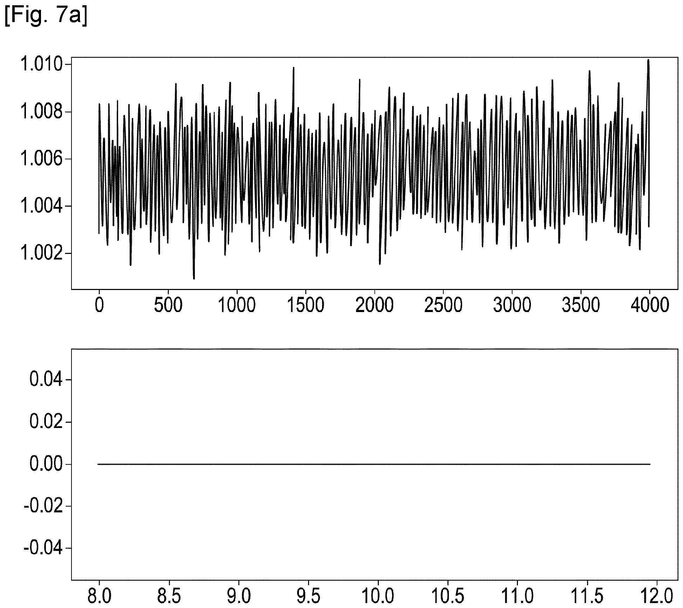

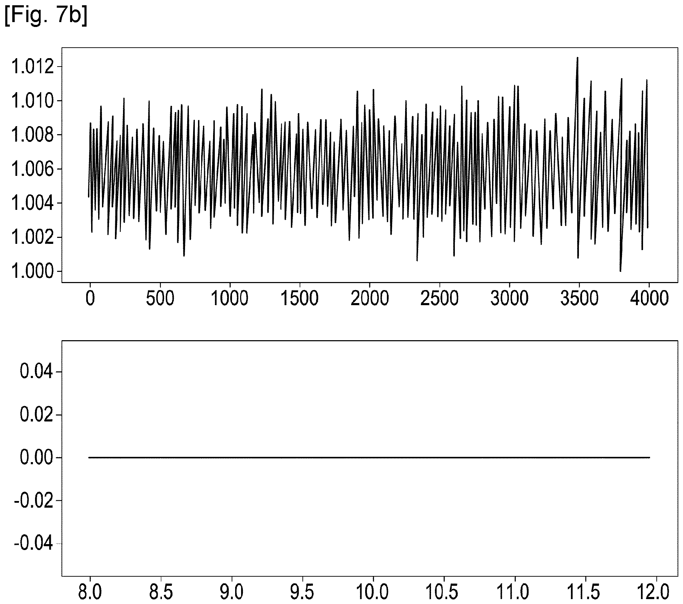

In a second use case, the device 101 is an eyewear, and the eyewear is placed in a table. The figures 7-a and 7-b represent in two situations of the second use case, the signal representing vibrations received by the sensor 103 (top part) and the power spectrum of this signal (bottom part).

In both situations, the power spectrum of the signal has been threshold. In both cases no peak has been detected and one determines that the eyewear is not worn by the user.

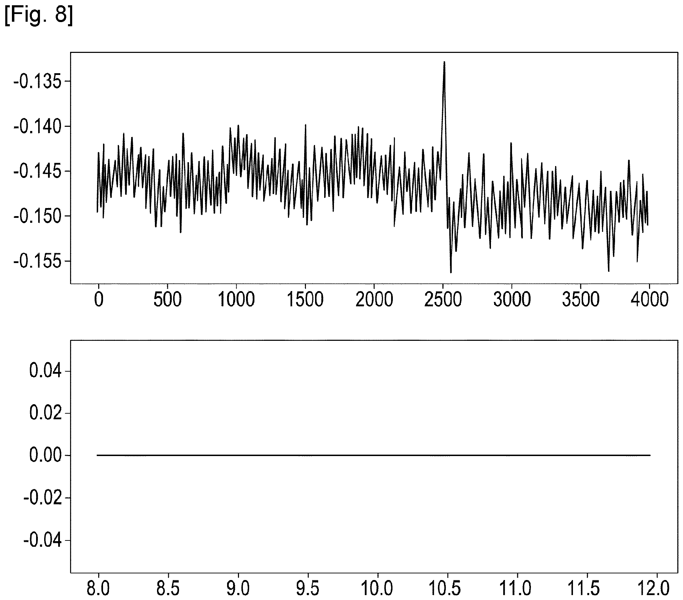

In a third use case, the device 101 is an eyewear, and the eyewear is placed in a collar of a clothe worn by the user. The user is immobile. The figure 8 represents the signal representing vibrations received by the sensor 103 (top part) and the power spectrum of this signal (bottom part).

One can observe that the signal is more structured than in the use second case, however after applying a threshold to the power spectrum of the signal no peak has been detected. One determines that the eyewear is not worn by the user.

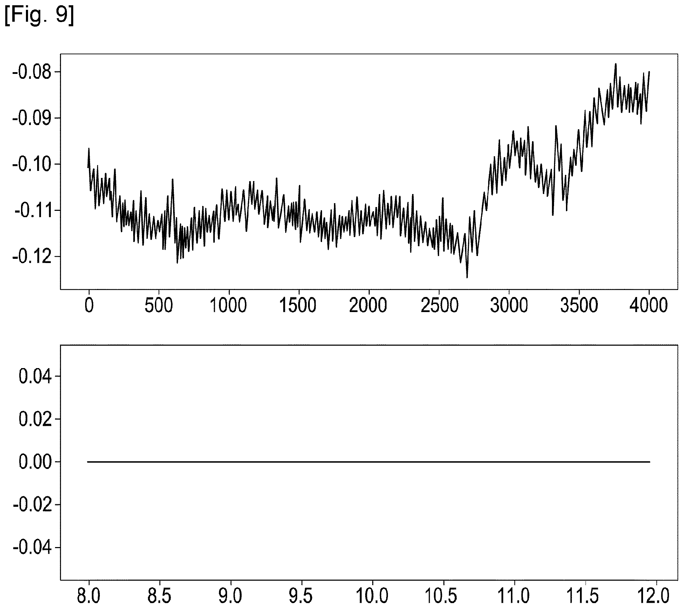

In a fourth use case, the device 101 is an eyewear, and the eyewear is placed in a backpack worn by the user. The user is immobile. The figure 9 represents the signal representing vibrations received by the sensor 103 (top part) and the power spectrum of this signal (bottom part).

One can observe that the signal is more structured than in the second use case, however after applying a threshold to the power spectrum of the signal no peak has been detected. One determines that the eyewear is not worn by the user.

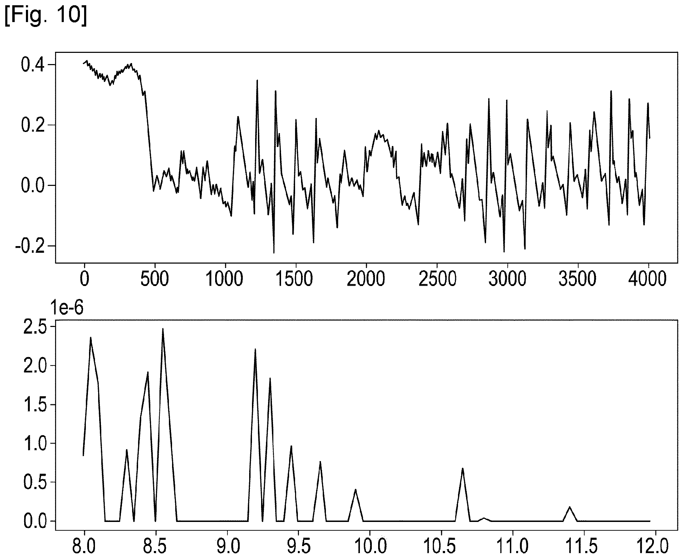

In a fifth use case, the device 101 is an eyewear, and the eyewear is placed in a backpack worn by the user. The user is walking. The figure 10 represents the signal representing vibrations received by the sensor 103 (top part) and the power spectrum of this signal (bottom part).

One can observe that the signal representing the vibrations comprises a plurality of peaks, each peak representing a step.

Regarding the spectral power, several peaks are above the predetermined threshold. Therefore, using the criteria of the presence of peaks in the spectral power, one would inaccurately determine that the user is wearing the eyewear. As the threshold power spectrum comprises too many powerful peaks, using the same approach as in static condition could lead to wrong prediction.

In this case, one may use advantageously the signal-to-noise ratio of the power spectrum or the peak signal-to-noise ratio of the power spectrum, to determine if the user is wearing the eyewear. In this fifth use case, the signal-to-noise ratio is -0.43 db and the peak signal-to-noise ratio of the power spectrum is 9.4 db. Using the criteria related to the signal-to-noise ratio and the peak signal-to-noise ratio (SNR > 0 or pSNR > 13 if wearing) one determines that the user is not wearing the eyewear.

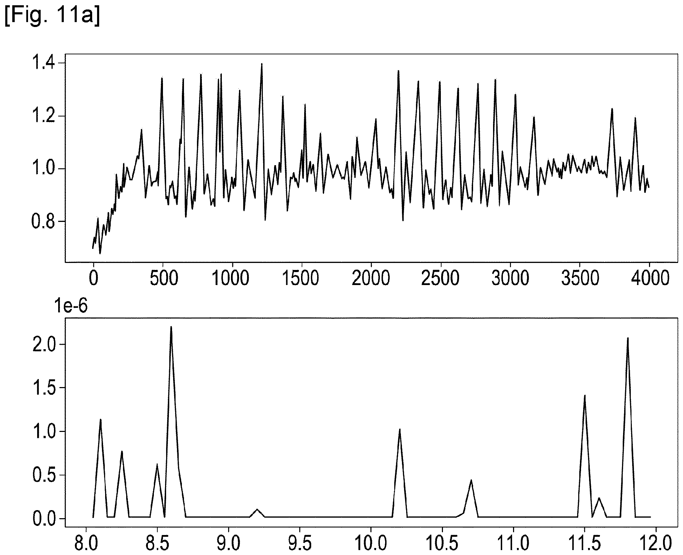

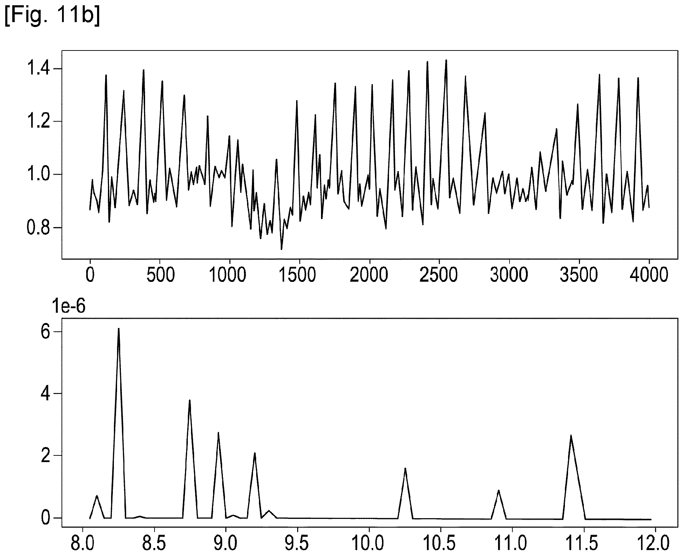

In a sixth use case the device 101 is an eyewear, and the eyewear is worn in a regular way by the user. The user is walking. The figure 11-a and 11-b represents for two situations the signal representing vibrations received by the sensor 103 (top part) and the power spectrum of this signal (bottom part).

In the first situation, the signal to noise ratio of the power spectrum is 0.98 dB and the peak signal to noise ratio of the power spectrum is 9.7 dB. Therefore, the condition of having a signal to noise ratio of the power spectrum below 0 dB is achieved, and one accurately determines that the user is wearing the eyewear.

In the second situation, the signal to noise ratio of the power spectrum is -0.31 dB and the peak signal to noise ratio of the power spectrum is 13.02 dB. Therefore, the condition of having a peak signal to noise ratio of the power spectrum above 13.02 dB is achieved, and one accurately determines that the user is wearing the eyewear.

**Claims**

1. Device (101) intended to be worn by a user, the device (101) being configured for determining if the user is wearing the device (101), the device (101) being configured:

   - to obtain a signal representing vibrations produced by the user,
   - to determine, in a predetermined frequency range, a power spectrum of the signal,
   - to determine, based on the power spectrum, if the user is wearing the device (101),

   the predetermined frequency range being from 8 Hz to 12 Hz.

2. The device (101) according to claim 1, the device (101) being configured to detect peaks in the power spectrum and determine that the user is wearing the device (101) when at least one peak is detected.

3. The device (101) according to claim 2, the device (101) being configured to compare at least one value of the power spectrum to a predetermined level and to detect a peak when at least one value of the power spectrum is above the predetermined level.

4. The device (101) according to any one of the claims 1 to 3, the device (101) being configured to determine a signal-to-noise ratio of the power spectrum and to determine, based on the signal-to-noise ratio, that the user is wearing the device (101).

5. The device (101) according to claim 4, the device (101) being configured to determine that the user is wearing the device (101) when the signal-to-noise ratio is above a predetermined signal-to-noise ratio threshold.

**6.** The device (101) according to claim 5, the predetermined signal-to-noise ratio threshold being 0dB.

**7.** The device (101) according to any one of the claims 1 to 5, the device (101) being configured to determine a peak signal-to-noise ratio of the power spectrum and to determine, based on the peak signal-to-noise ratio, that the user is wearing the device (101).

**8.** The device (101) according to claim 7, the device (101) being configured to determine that the user is wearing the device (101) when the peak signal-to-noise ratio is above a predetermined peak signal-to-noise ratio threshold.

**9.** The device (101) according to claim 8, the predetermined peak signal-to-noise ratio threshold being 13dB.

**10.** The device (101) according to any one of the claims 1 to 9, the device (101) comprising a sensor (103) for generating the signal representing the vibrations.

**11.** Head-mountable device, the head-mountable device being configured for determining if a user is wearing the head-mountable device, the head-mountable device being configured:

- to obtain a signal representing vibrations produced by the user,
- to determine, in a predetermined frequency range, a power spectrum of the signal,
- to determine, based on the power spectrum, if the user is wearing the head-mountable device,

the predetermined frequency range being from 8 Hz to 12 Hz.

**12.** The head-mountable device according to claim 11, the head-mountable device comprising a sensor (103) for generating the signal representing the vibrations.

**13.** Eyewear (EY), the eyewear (EY) being configured for determining if a user is wearing the eyewear (EY), the eyewear (EY) being configured:

- to obtain a signal representing vibrations produced by the user,
- to determine, in a predetermined frequency range, a power spectrum of the signal,
- to determine, based on the power spectrum, if the user is wearing the eyewear (EY),

the predetermined frequency range being from 8 Hz to 12 Hz.

**14.** The eyewear (EY) according to claim 13, the eyewear (EY) comprising a sensor (103) for generating the signal representing the vibrations.

**15.** Computer implemented method for determining when a device (101), intended to be worn by a user, is worn by the user, the computer implemented method comprising:

- obtaining (501) a signal representing vibrations produced by the user,
- determining, (502) in a predetermined frequency range, a power spectrum of the signal,
- determining (503), based on the power spectrum, if the user is wearing the device (101), the predetermined frequency range being from 8 Hz to 12 Hz.

[Fig. 1]

101
103
102
102-a
102-b

[Fig. 2]

EY
H2
F
103
R1
A2
R2
H1
L2
B
A1
L1
F1

[Fig. 3]

301

103

[Fig. 4]

ARE

H2
F
103
R2
A2
R1
SCR
H1
L2
B
A1
L1
F1

[Fig. 5]

[Fig. 6a]

[Fig. 6b]

[Fig. 7a]

[Fig. 7b]

[Fig. 8]

[Fig. 9]

[Fig. 10]

[Fig. 11a]

[Fig. 11b]

Europäisches
Patentamt

European
Patent Office

Office européen
des brevets

# EUROPEAN SEARCH REPORT

**Application Number**

EP 23 30 7134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2015/057967 A1 (ALBINALI FAHD KHALAF [US]) 26 February 2015 (2015-02-26) * par. [0091]; paragraph [0032] - paragraph [0039]; figure 1A * | 1-15 | INV. G02C11/00 A61B5/00 G06F1/16 G02B27/01 G06F1/3231 |
| A | US 2020/367789 A1 (MOFFAT GRAEME DANIEL [CA] ET AL) 26 November 2020 (2020-11-26) * the whole document * | 1-15 | |
| A | US 2023/104773 A1 (GURIN ILYA [US] ET AL) 6 April 2023 (2023-04-06) * paragraph [0066] - paragraph [0069]; figure 4 * | 1-15 | |
| A | US 2023/258963 A1 (DROBE BJORN [SG] ET AL) 17 August 2023 (2023-08-17) * the whole document * | 1-15 | |

**TECHNICAL FIELDS SEARCHED (IPC)**

G02C
A61B
G02B
G06F

The present search report has been drawn up for all claims

| Place of search | Date of completion of the search | Examiner |
|---|---|---|
| Munich | 13 May 2024 | Klein, Thomas |

EPO FORM 1503 03.82 (P04C01)

**ANNEX TO THE EUROPEAN SEARCH REPORT
ON EUROPEAN PATENT APPLICATION NO.**

EP 23 30 7134

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2024

| Patent document cited in search report | Publication date | Patent family member(s) | Publication date |
|---|---|---|---|
| US 2015057967 A1 | 26-02-2015 | NONE | |
| US 2020367789 A1 | 26-11-2020 | CA 3087592 A1 | 11-07-2019 |
| | | US 2020367789 A1 | 26-11-2020 |
| | | WO 2019134043 A1 | 11-07-2019 |
| US 2023104773 A1 | 06-04-2023 | NONE | |
| US 2023258963 A1 | 17-08-2023 | AU 2021313356 A1 | 16-02-2023 |
| | | BR 112023000882 A2 | 07-02-2023 |
| | | CN 116133577 A | 16-05-2023 |
| | | EP 4185919 A1 | 31-05-2023 |
| | | JP 2023537682 A | 05-09-2023 |
| | | KR 20230042296 A | 28-03-2023 |
| | | US 2023258963 A1 | 17-08-2023 |
| | | WO 2022018080 A1 | 27-01-2022 |

EPO FORM P0459

For more details about this annex : see Official Journal of the European Patent Office, No. 12/82